# DEMANDE DE BREVET EUROPEEN

(11) **EP 1 034 739 A1**
(43) Date de publication de la demande: **13.09.2000**
(21) Numéro de dépôt: 00400553.4
(22) Date de dépôt: 01.03.2000
(51) Int. Cl.: A61B 5/103, G01N 19/02

(54) **Dispositif et prodédé de caractérisation de la peau par tribométrie**

(30) Priorité: 11.03.1999 FR 9903010
(71) Demandeur: CENTRE TECHNIQUE DES INDUSTRIES MECANIQUES, F-60300 Senlis (FR)
(72) Inventeur: Chen, Yaming, 60300 Senlis (FR); Pavy, Jean-Claude, 60300 Senlis (FR)
(74) Mandataire: Bertrand, Didier

(57) **Abrégé**

Ce dispositif de caractérisation de la peau humaine comprend un tribomètre 2, 5, 7, 8 comportant un bras de mesure 1 à une première extrémité duquel sont montés une pointe de mesure et un capteur de mesure 3 susceptible de mesurer une force de frottement s'exerçant sur la pointe, et dont la seconde extrémité est montée dans un support 2 permettant un déplacement linéaire de la première extrémité. Un amplificateur 7 et des moyens de traitement 8 et de visualisation 9 de signal sont associés au capteur.

## Description

La présente invention concerne la caractérisation de la peau humaine.

Il existe de nombreuses méthodes pour tenter de caractériser la peau humaine, par la mesure de certaines propriétés, comme l'élasticité, la comprimabilité, l'hygrométrie, etc. Par exemple, le document US 4 206 769 A décrit une sonde de mesure de telles propriétés.

On connaît aussi par le document FR 2 749 793 un rasoir électrique qui comporte un capteur de friction intégré, mesurant en permanence le degré de friction qui apparaît entre la peau et les grilles du rasoir. Ce résultat est exploité pour modifier le niveau de rasage, mais nullement pour caractériser la peau humaine.

Cependant, ces méthodes le plus souvent conçues pour des applications limitées à la cosmétique, rendent compte seulement de certains aspects de la peau. Le but de l'invention est d'explorer une autre façon de caractériser la peau.

Ce but est atteint selon l'invention par un dispositif de caractérisation de la peau humaine, caractérisé en ce qu'il comprend un tribomètre, c'est-à-dire un appareil de mesure de la force de frottement entre deux surfaces antagonistes, et par le procédé associé, qui consiste à mesurer au moyen d'un tribomètre la force de frottement entre la peau et une pointe de mesure se déplaçant à la surface de la peau. La vitesse de déplacement est de préférence comprise entre 0,1 mm/min et 100 mm/min.

Avantageusement, le tribomètre comprend un bras de mesure à une première extrémité duquel sont montés une pointe de mesure et un capteur de mesure susceptible de mesurer une force de frottement s'exerçant sur la pointe, et dont la seconde extrémité est montée dans un support permettant un déplacement linéaire de la première extrémité.

Avantageusement, la première extrémité comporte une tige terminale articulée, de préférence selon au moins deux axes non parallèles, permettant à la pointe de suivre librement la surface explorée de la peau d'un sujet.

Avantageusement, le bras comporte au moins une masse réglable, par exemple au moyen de masselottes qu'on ajoute à volonté et/ou de masses coulissantes, permettant d'exercer une charge comprise de préférence entre 0,1 N et 10 N.

Avantageusement, la pointe de mesure est à bout hémisphérique, avec un rayon compris par exemple entre 5 et 200 mm.

De manière préférentielle, la pointe de mesure est couverte d'un tissu, par exemple par collage, notamment d'un tissu autocollant. Ce tissu peut être constitué par tout type de matériau tissé à base de fibres organiques d'origine végétale, animale, minérale ou de synthèse. Un matériau tissé présente l'avantage d'avoir une faible différence entre le coefficient de frottement statique et le coefficient de frottement dynamique.

Le dispositif comprend encore, en aval du capteur, un amplificateur qui reçoit les signaux du capteur, notamment des signaux de charge et de force de frottement, et les envoie sur un dispositif de visualisation ou d'archivage.

D'autres caractéristiques et avantages de la présente invention ressortiront de la description ci-après d'un exemple de réalisation de l'invention illustré dans les dessins annexés sur lesquels :
- la figure 1 est un schéma du dispositif conforme à l'invention,
- la figure 2 est une vue de côté du détail de réalisation du bras de mesure du dispositif de la figure 1,
- la figure 3 est une vue en coupe III-III du bras de la figure 2.
- Les figures 4 et 5 sont deux graphes de la force de frottement obtenue lors de tests sur deux sujets différents.

Le dispositif de l'invention comporte essentiellement un bras de mesure 1 porté par un support 2 à moteur permettant de réaliser un mouvement linéaire dont la course est de longueur réglable. Le bras de mesure 1 comporte à son extrémité un capteur de force piézo-électrique tridimensionnel 3 représenté posé par l'intermédiaire d'une pointe de mesure sur le bras 4 d'une personne, sous une charge réglable 5. Une ligne 6 envoie les signaux émis par le capteur 3, représentatifs de la charge et de la force de frottement, vers un amplificateur 7 puis vers un micro-ordinateur 8, en vue de l'exploitation, de l'archivage et/ou de l'affichage des résultats (sur écran 9, sur traceur, etc.)

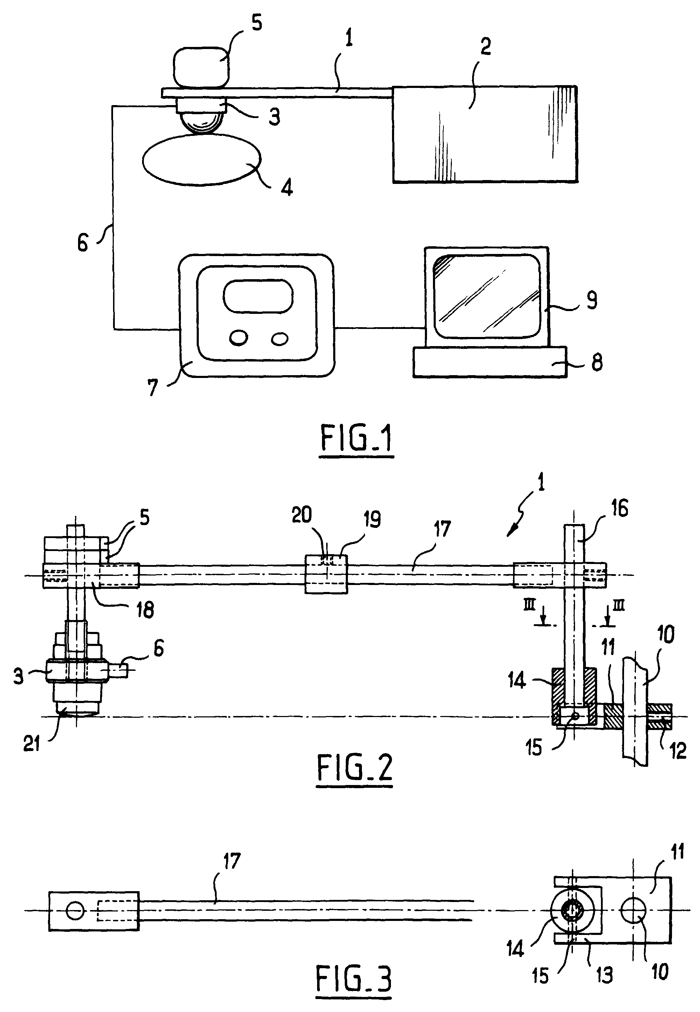

Les figures 2 et 3 montrent le détail d'un exemple de réalisation de l'extrémité du bras de mesure 1. Il comprend une première partie terminée par une broche verticale 10, cette partie étant portée par le support 2 de façon à être mobile en translation. Sur cette broche 10, une chape 11 vient se fixer grâce à une vis 12 à une hauteur réglable. Dans la fourche 13 de la chape 11, un culot 14 est fixé de manière pivotante grâce à des axes 15, le culot 14 portant une broche tournante 16 sur laquelle se fixe à une hauteur réglable une tige 17 globalement horizontale (mais pouvant s'incliner grâce à l'articulation du culot 14). La tige 17 porte à son extrémité un embout 18 sur lequel sont montés d'une part le capteur 3 et d'autre part une charge fixe 5, par exemple de 20g. Le long de la tige 17, une charge mobile, par exemple de 20g également, peut coulisser et être maintenue en position par une vis 20. L'ensemble est très léger (moins de 150 g dans un exemple de réalisation). Le capteur utilisé 3 est, dans l'exemple de réalisation, un capteur Kistler 9251A ; la pointe de mesure 21 est cylindrique à bout hémisphérique (rayon de 30 mm), et vient sensiblement au niveau de l'axe d'articulation 15 quand la tige 17 est horizontale. La vitesse d'avance du support motorisé 2 est par exemple de 0,05 mm/s pendant environ 40 s.

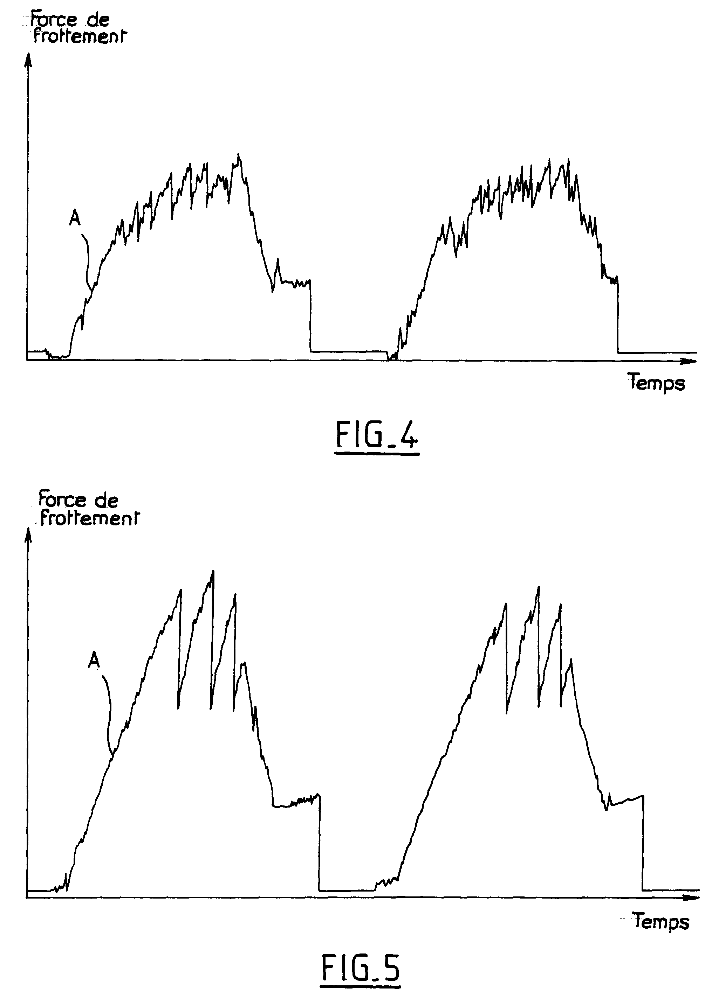

Des tests ont été effectués sur l'avant-bras de sujets. Pour les tests, la pointe 21 du capteur était couverte d'une pièce de tissu collé, servant de surface antagoniste au bras du sujet. On a enregistré sur des graphes, analogues à ceux des figures 4 et 5, le signal de la force de frottement en fonction du temps (et donc du déplacement de la pointe à la surface de la peau).

On a noté que la variation de force de frottement durant l'essai est très variable en fonction de la personne, mais très reproductible pour une personne donnée, et donc caractéristique de cette personne. On trouve d'ailleurs que l'évolution de force de frottement est identique lorsqu'on effectue le même test sur la même personne deux jours après le premier test.

La première partie du signal (A sur les figures 4 et 5) de la force de frottement correspond à une déformation élastique de l'ensemble structurel constitué notamment de la peau et des muscles. On note le coefficient de frottement statique lors du premier mouvement entre la peau et le point de mesure. La suite du signal contient à la fois le coefficient de frottement statique et le coefficient de frottement dynamique.

L'amplitude et la fréquence du signal, qui sont très personnalisées, peuvent contenir des informations liées à la personne, à la texture de la peau, aux poils, à la graisse, au comportement en cisaillement de la peau et de la structure musculaire.

Le dispositif et le procédé de l'invention peuvent permettre notamment de déceler des anomalies du profil habituel d'un sujet (notamment consécutives à des maladies cutanées non nécessairement décelables à l'oeil nu), ou le retour à un profil habituel, et donner des indications comparables à celles qu'un médecin expérimenté peut déduire quand il passe la main sur la peau d'un patient.

## Revendications

1. Dispositif de caractérisation de la peau humaine, caractérisé en ce qu'il comprend un tribomètre (2, 5, 7, 8) comportant une pointe de mesure (21) agencée pour se déplacer à la surface de la peau.

2. Dispositif selon la revendication 1, caractérisé en ce que le tribomètre comprend un bras de mesure (1) à une première extrémité duquel sont montés ladite pointe de mesure (21) et un capteur de mesure (3) susceptible de mesurer une force de frottement s'exerçant sur la pointe (21), et dont la seconde extrémité est montée dans un support (2) permettant un déplacement linéaire de la première extrémité.

3. Dispositif selon la revendication 2, caractérisé en ce que la première extrémité comporte une tige terminale (17) articulée.

4. Dispositif selon la revendication 3, caractérisé en ce que la tige (17) est articulée selon au moins deux axes non parallèles (15, 16).

5. Dispositif selon l'une quelconque des revendications 2 à 4, caractérisé en ce que le bras (1) comporte au moins une masse réglable (5, 19).

6. Dispositif selon l'une quelconque des revendications 2 à 5, caractérisé en ce que la pointe de mesure (21) est à bout hémisphérique.

7. Dispositif selon l'une quelconque des revendications 2 à 6, caractérisé en ce que la pointe de mesure (21) est couverte d'un tissu.

8. Dispositif selon l'une quelconque des revendications 2 à 7, caractérisé en ce que le capteur (3) envoie ses signaux à un amplificateur (7) qui les envoie sur un dispositif de traitement (8) associé à des moyens de visualisation (9) ou d'archivage.

9. Procédé de caractérisation de la peau humaine, caractérisé en ce qu'il consiste à mesurer au moyen d'un tribomètre (2, 5, 7, 8) la force de frottement entre la peau et une pointe de mesure (21) se déplaçant à la surface de la peau.
